**Europäisches Patentamt**

**European Patent Office**

**Office européen des brevets**

(19)

(11) Veröffentlichungsnummer: **0 144 877**

**A2**

(12) # EUROPÄISCHE PATENTANMELDUNG

(21) Anmeldenummer: **84114204.5**

(22) Anmeldetag: **24.11.84**

(51) Int. Cl.⁴: **A 61 H 33/04**

(30) Priorität: **02.12.83 DE 3343664**

(43) Veröffentlichungstag der Anmeldung:
**19.06.85 Patentblatt 85/25**

(84) Benannte Vertragsstaaten:
**AT BE CH DE FR LI LU NL**

(71) Anmelder: **Gembrys, Paul R.**
**Am Taubergrund 22**
**D-6990 Bad Mergentheim-Edelfingen(DE)**

(72) Erfinder: **Gembrys, Paul R.**
**Am Taubergrund 22**
**D-6990 Bad Mergentheim-Edelfingen(DE)**

(74) Vertreter: **Tergau, Enno et al,**
**Hefnersplatz 3 Postfach 11 93 47**
**D-8500 Nürnberg 11(DE)**

(54) **Vorrichtung zur therapeutischen Temperierung von Körperpartien.**

(57) Eine Vorrichtung zur therapeutischen Temperierung von Körperpartien oder auf diese aufgelegte Zwischenlagen, z.B. therapeutische Fango- und/oder Paraffinpackungen, enthält ein auf die Körperpartie oder die Zwischenlage auflegbares, biegeelastische Temperaturübertragungselement (3), das aus einem von mindestens einem Flüssigkeitskanal (7) durchsetzten Auflegelappen (8) besteht. Ein Versorgungsteil (1) für die Flüssigkeit enthält zwei Flüssigkeitsbehälter (20,21), wobei der eine mit einer Heizung (22), der andere mit einer Kühlvorrichtung (24) versehen ist. Beide Flüssigkeitsbehälter (20,21) sind über das elektrisch steuerbare Ventil (29) mit der Umwälzpumpe (31) verbunden. Der Steuerteil (2) der Vorrichtung enthält eine elektrische Schaltung, die aus einer Komparatorschaltung (Prozessor 40), einem Speicher (42), einem Taktgeber (41), einer Eingangsschaltung (45) und einer Ausgangsschaltung (48) besteht. Im Speicher (42) sind mehrere Vorwahltemperaturen abspeicherbar, so daß die Temperatur der Flüssigkeit in zeitlich aufeinanderfolgenden Intervallen auf diese Vorwahltemperaturen regelbar ist.

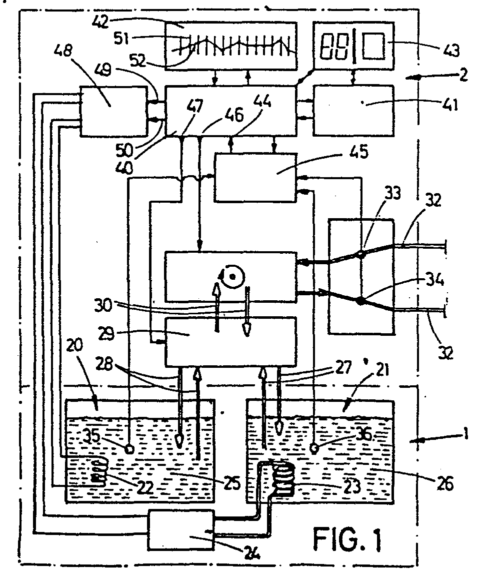

FIG.1

EP 0 144 877 A2

**TERGAU & POHL**
PATENTANWÄLTE
HEFNERSPL. 3 · POSTF. 11 93 47
**8500 NÜRNBERG 11**

Paul R. Gembrys, Am Taubergrund 22,
6990 Bad Mergentheim

Vorrichtung zur therapeutischen Temperierung
von Körperpartien

Die Erfindung betrifft eine Vorrichtung zur therapeutischen Temperierung von Körperpartien oder auf diese aufgelegten Zwischenlagen, z.B. therapeutischen Fango- und/oder Paraffinpackungen nach dem Oberbegriff des Anspruches 1.

Im Bereich der physikalischen Therapie ist es bekannt, Körperteile mit Auflagen oder sogenannten Packungen zur Kühlung oder Erwärmung zu versehen. Dadurch werden vermittels der Wärme- oder Kälteeinwirkung Körperreaktionen hervorgerufen, die sich beispielsweise sowohl auf Durchblutung als auch auf die Psyche des Patienten vorteilhaft auswirken. So werden Moorbäder oder Moorpackungen am menschlichen Körper oder einzelne Gliedmassen zur Wärmebehandlung bei inneren Krankheiten, akuten Entzündungen, Nervenkrankheiten usw. appliziert.

Um beispielsweise die zeitliche Konstanz des Temperaturverlaufes solcher Packungen noch weiter zu verbessern, ist man in der Vergangenheit bereits dazu übergegangen, sogenannte Moor/Paraffin- oder Paraffinpackungen zu applizieren.

Ein bekanntes "Hausmittel" zur Temperierung von Körperteilen und/oder den genannten Packungen ist das sogenannte Heizkissen, das entweder unmittelbar auf die Körperpartien aufgelegt oder um die Packungen herumgelegt werden kann. Eine Regelung der Kissen- bzw. Packungstemperatur mit zeitlich und lokal hoher Konstanz über elektrische Heizelemente in Verbindung mit im Kissen wirksamen Thermostaten ist jedoch nur unter Aufwendung eines sehr hohen technischen Aufwandes möglich und führt nur zu einem minderen Erfolg.

Als Verbesserung wurde in der Vergangenheit bereits vorgeschlagen (US-PS 39 67 627), eine wärmeübertragende Flüssigkeit über ein Leitungssystem von einem beheiz- oder kühlbaren Behälter durch ein auf die zu temperierenden Körperpartien auflegbares, biegeelastisches Wärmeübertragungselement zu pumpen. Damit war es zwar möglich, physikalische Therapie mit Wärme oder Kälte durchzuführen, doch ein beliebig einstellbarer Temperaturverlauf zur Erzielung therapeutischer Effekte war damit nicht möglich.

Die europäische Patentschrift EP-PS 39 443 beschreibt eine Vorrichtung zur Kühlung von Körperteilen, die einen elektronischen Speicher zur Eingabe einer konstanten Behandlungstemperatur bzw. einer Behandlungsendtemperatur aufweist, die durch kontinuierliche Annäherung erreicht wird. Eine Wärmetherapie ist mit dieser Vorrichtung ebensowenig durchführbar, wie eine Therapie mit beliebig festlegbarem Temperaturverlauf, um Körperpartien beispielsweise einer alternierenden Temperatur, Temperaturstürzen oder -schüben auszusetzen.

Der Erfindung liegt die Aufgabe zugrunde, eine Vorrichtung zur therapeutischen Temperierung von Körperpartien zu schaffen, bei der der Temperaturverlauf universell in aufeinanderfolgenden Zeitintervallen zu quasi beliebigen Temperaturen hin steuerbar ist. Dabei sollen im wesentlichen konstante Temperaturverläufe genauso realisierbar sein, wie mehr oder weniger stark alternierende.

Diese Aufgabe wird gemäß den kennzeichnenden Merkmalen des Anspruches 1 gelöst. Da in den beiden Flüssigkeitsbehältern jeweils eine Heizung bzw. eine Kühlvorrichtung vorgesehen ist, weisen diese gegebenenfalls stark unterschiedliche Temperaturen auf. Zur Temperaturänderung der Übertragungsflüssigkeit braucht damit kein Heiz- oder Kühlvorgang in Gang gesetzt werden, sondern nur das Umschalt-Mischventil zwischen beiden Behältern entsprechend betätigt werden. Je nach Umschaltdauer des Ventils können somit schnelle Temperaturänderungen im Auflegelappen vorgenommen werden.

Vorteilhafterweise kann also mit der erfindungsgemäßen Vorrichtung die Temperatur beispielsweise einer Packung nicht nur über einen langen Zeitraum wirklich konstant gehalten werden (das Flüssigkeitssystem reagiert aufgrund seines relativ großen Wärmeinhaltes relativ träge auf Temperaturschwankungen), es ist darüber hinaus auch ein gleichmäßiges Anheben oder Absenken der Packungstemperatur über einen vorzugebenden Zeitraum möglich. Weiterhin können mit der Vorrichtung auf die zu behandelnden Körperpartien gezielt zeitlich bestimmte Temperaturverläufe, -schübe oder -stürze einwirken, wie sie sich zur Behandlung von unterschiedlichsten Krankheiten als optimal herausgestellt haben. Dabei sind keinerlei elektrische Meß- oder Regelvorrichtungen am Patientenkörper (oder dem Temperaturübertragungselement) notwendig, so daß die Sicherheit des Patienten vor Stromschlag erheblich verbessert ist. Dabei ist zu bedenken, daß Packungen oftmals in Baderäumen appliziert werden, in welchen mit elektrischem Gerät jeglicher Art höchst sorgsam umgegangen werden muß.

Wichtig ist weiterhin, daß durch den Einbau eines Speichers für die Vorwahltemperaturen der Therapieverlauf bereits vor seinem Start festgelegt werden kann. Dadurch ist es nicht notwendig, daß während der gesamten Behandlungsdauer das Bedienungspersonal anwesend ist.

Gemäß dem kennzeichnenden Merkmal des Anspruches 2 sind die gewünschten Vorwahltemperaturen von Hand vorwählbar. Dafür sind verschiedene Bedienungselemente denkbar. Die kennzeichnenden Merkmale der Ansprüche 4 und 5 beschreiben in diesem Zusammenhang eine besonders vorteilhafte, auch für ungeschultes Personal sehr einfach zu bedienende Bauform für die Temperaturvorwahl. Besonders einfach und übersichtlich wird die Bedienung des Speichers, wenn er eine Mehrzahl von gesonderten Temperatureinstellelementen aufweist, von denen jedes einem bestimmten Zeitintervall zugeordnet ist. Sind die Temperatureinstellelemente als parallel nebeneinanderliegende Temperaturschieber in Form von Schiebepotentiometern ausgebildet, so wird die Bedienung der Vorrichtung auch für Nichtfachkräfte besonders einfach und übersichtlich, da der zeitliche Verlauf der Temperatur in Kurvenform unmittelbar von den Schieberstellungen ablesbar ist.

Durch den Einsatz eines Umschalt-Mischventils entsprechend dem Kennzeichnungsmerkmal des Anspruches 3 kann durch die von der elektrischen Schaltung gesteuerten Betätigung dieses Ventils die Therapie-Temperatur besonders einfach geregelt werden.

Die Erfindung ist anhand eines Ausführungsbeispieles in der Zeichnung näher erläutert. Es zeigen:

Fig. 1 einen schematischen Aufbau der wesentlichen Komponenten der Vorrichtung,

Fig. 2 eine schematische Darstellung des Wärmeübertragungselementes und

Fig. 3 einen Schnitt gemäß A-A in Fig. 2.

Die Vorrichtung besteht im wesentlichen aus einem vorzugsweise in einem Schrank oder Rack gemeinsam unterbringbaren Versorgungsteil 1 und Steuerteil 2, einem um die Körperpartie herumlegbaren biegeelastischen Temperaturübertragungselement 3 sowie zwischen letzterem und dem Versorgungs- und Steuerteil wirksamen Schlauchleitungen 4, nämlich einer Zuführungsleitung 5 und einer Rückführungsleitung 6.

Das Temperaturübertragungselement 3 besteht aus einem von mindestens einem Flüssigkeitskanal 7 gleichmäßig durchsetzten Auflagelappen 8 mit einer Kontaktanlegefläche 9, die die körperzugewandte Seite des Temperaturübertragungselements 3 bildet. Der Flüssigkeitskanal 7 ist in gutem Wärmekontakt auf der Rückseite der Kontaktanlegefläche 9 aufgebracht und wird von einer biegeelastischen Kontaktierungsmasse 10 umgeben. Die körperabgewandte Rückseite 11 des Temperaturübertragungselementes 3 ist mit einer Wärmeisolationsschicht 12 versehen.

Das fluidische Versorgungsteil 1 besteht aus zwei Flüssigkeitsbehältern 20,21, von denen der eine mit einer elektrischen Heizung 22, der andere mit einem Wärmetauscher 23 eines Kühlaggregates 24 verbunden ist. Die Flüssigkeit im Flüssigkeitsbehälter 20 ist somit eine Heizflüssigkeit 25, die im Flüssigkeitsbehälter 21 eine Kühlflüssigkeit 26. Über doppelte Rohrleitungen 27,28 stehen die Flüssigkeitsbehälter 20,21 mit einem elektrischen Umschalt-Mischventil 29 in fluidischer Verbindung, das über weitere Rohrleitungen 30 an eine Umwälzpumpe 31 angeschlossen ist. An diese sind, gegebenenfalls unter Zwischenschaltung weiterer Rohrabschnitte 32, die Schlauchleitungen 4 angeschlossen, die zum Temperaturübertragungselement 3 führen.

Die Temperatur im Flüssigkeitssystem wird durch Temperaturfühler 33,34 in der Zuführungsleitung 5 und der Rückführungsleitung 6 gemessen. Weitere Temperaturfühler 35,36 befinden sich in den Flüssigkeitsbehältern 20,21 und dienen insbesondere zur Konstanthaltung der Temperaturen der Heizflüssigkeit 25 und Kühlflüssigkeit 26 für den Fall, daß der Kreislauf durch Abschaltung der Umwälzpumpe 31 unterbrochen ist.

Die Vorrichtung wird über eine als speicherprogrammierbare Steuerung ausgelegte Schaltung betrieben, die im wesentlichen aus einem Prozessor 40, einem Taktgeber 41, einem manuell betätigbaren Speicher 42 sowie einer elektrooptischen Anzeigevorrichtung 43 besteht. Über einen ersten Eingang 44 steht der Prozessor 40 mit einer Eingangsschaltung 45 in Verbindung, in welcher beispielsweise der Mittelwert aus den Thermospannungen der Temperaturfühler 33,34 gebildet wird und/oder die Thermospannungen der weiteren Temperaturfühler 35,36 zur Aufschaltung auf den Eingang 44 aufbereitet werden. Über einen Ausgang 46 ist der Prozessor (gegebenenfalls unter Zwischenschaltung einer nicht dargestellten Leistungsstufe) mit der Umwälzpumpe 31 verbunden und regelt deren Förderleistung je nach Bedarf. Über einen weiteren Ausgang 47 des Prozessors 40 wird das Umschalt-Mischventil 29 geregelt, gegebenenfalls unter Zwischenschaltung einer weiteren nicht dargestellten Leistungsstufe. Die Heizung 22bzw. das Kühlaggregat 24 werden über eine Ausgangsschaltung 48 betrieben, die über Ausgangsleitungen 49,50 ebenfalls mit dem Prozessor 40 in Verbindung steht.

Getaktet wird der Prozessor 40 über den Taktgeber 41, der den nacheinander abfolgenden Zeitabschnitten zugeordneten Inhalt des Speichers 42 auf den Prozessor schaltet, wodurch die Ausgänge 46,47,49,50 in vorprogrammierter Weise betrieben werden. Die Anzeigevorrichtung 43 dient sowohl zur Anzeige der jeweiligen mittleren Temperatur (Mittelwert der Thermospannungen aus den Temperaturfühlern 33,34) als auch beispielsweise der noch verbleibenden Behandlungszeit.

Der Speicher 42 ist mit einer Mehrzahl von Temperatureinstellelementen 51 versehen, die als parallel neben- einander liegende Schiebepotentiometer (Temperatur- schieber) ausgebildet sind und mit denen sich besonders leicht eine zeitliche Temperaturkurve 52 darstellen läßt.

Der auf der Kontaktanlegefläche 9 angeordnete Flüssigkeitskanal 7 ist ein biegsamer Schlauch 55, der zulauf- seitig eine Mehrfachverzweigung 56 aufweist und mehrere parallellaufende Einzelkanäle 57 bildet, die mit ihren rücklaufseitigen Enden 58 gemeinsam in die Rücklaufleitung 6 münden. Die Einzelkanäle 57 sind so zickzackförmig gelegt, daß sie in parallelen geraden Abschnitten einen wesentlichen Teil der Breite der Kontaktauflegefläche 9 überdecken.

Bezugszeichenliste

1 Versorgungsteil
2 Steuerteil
3 Temperaturübertragungselement
4 Schlauchleitungen
5 Zuführungsleitung
6 Rückführungsleitung
7 Flüssigkeitskanal
8 Auflagelappen
9 Kontaktanlegefläche
10 Kontaktierungsmasse
11 Rückseite
12 Wärmeisolationsschicht
20 Flüssigkeitsbehälter
21 "
22 Heizung
23 Wärmetauscher
24 Kühlaggregat
25 Heizflüssigkeit
26 Kühlflüssigkeit
27 Rohrleitung
28 "
29 Umschalt-Mischventil
30 weitere Rohrleitungen
31 Umwälzpumpe
32 Rohrabschnitte
33 Temperaturfühler
34 "
35 weitere Temperaturfühler
36 "      "
40 Prozessor
41 Taktgeber
42 Speicher
43 Anzeigevorrichtung
44 Eingänge
45 Eingangsschaltung
46 Ausgang
47 weiterer Ausgang
48 Ausgangsschaltung

49 Ausgangsleitung
50 Ausgangsleitung
51 Temperatureinstellelement
52 Temperaturkurve
55 Schlauch
56 Mehrfachverzweigung
57 Einzelkanäle
58 Enden

**TERGAU & POHL**
PATENTANWÄLTE
HEFNERSPL. 3 · POSTF. 11 93 47
8500 NÜRNBERG 11

Paul R. Gembrys, Am Taubergrund 22,
6990 Bad Mergentheim

Ansprüche

1. Vorrichtung zur therapeutischen Temperierung von Körperpartien oder auf diese aufgelegte Zwischenlagen, z.B. therapeutische Fango- und/oder Paraffinpackungen, mit

   1. einem auf die Körperpartie oder die Zwischenlage auflegbaren, biegeelastischen Wärmeübertragungselement (3), das

      1.1. aus einem Auflegelappen (8), der

      1.2. von mindestens einem Flüssigkeitskanal (7) gleichmäßig durchsetzt ist, besteht;

   2. einem Flüssigkeitsbehälter (20), der

      2.1. mit einer Heizung (22) und

      2.2. einer Kühlvorrichtung (24) versehen ist;

   3. einer Umwälzpumpe (31), die

      3.1. den Flüssigkeitsbehälter (20),

      3.2. mit dem Flüssigkeitskanal (7)

      3.3. über ein elektrisch stellbares Ventil (29) verbindet;

   4. einer elektrischen Schaltung, die aus

      4.1. einem Prozessor (40)

      4.2. einem Speicher (42),

      4.3. einem Taktgeber (41),

      4.4. einer Eingangsschaltung (45), die die von Temperaturfühlern (33,34,35,36) abgeleiteten, der Flüssigkeitstemperatur entsprechenden Signale aufbereitet und

4.5. einer Ausgangsschaltung (48), die aufgrund von elektrischen Signalen des Prozessors (40) die Heizung (22), das Kühlaggregat (24), das Ventil (29) und die Umwälzpumpe (31) steuert, besteht, wobei

4.6. eine feste, vom Benutzer vorwählbare Temperatur der Flüssigkeit im Speicher (42) abgespeichert wird;

4.7. die Temperatur der Flüssigkeit mittels der Heizung (22), der Kühlvorrichtung (24) und/ oder der Förderleistung der Umwälzpumpe (31) auf die vorgewählte Temperatur regelbar ist;

dadurch gekennzeichnet, daß

5. im Speicher (42) mehrere Vorwahltemperaturen abspeicherbar sind, so daß die Temperatur der Flüssigkeit in zeitlich aufeinanderfolgenden Intervallen auf diese Vorwahltemperaturen regelbar ist;

6. zwei Flüssigkeitsbehälter (20,21) vorgesehen sind, wobei

6.1. der eine mit einer Heizung (22),

6.2. der andere mit einer Kühlvorrichtung (24) versehen ist;

6.3. beide Flüssigkeitsbehälter (20,21) über das Ventil (29) mit der Umwälzpumpe (31) verbunden sind.

2. Vorrichtung nach Anspruch 1,

dadurch gekennzeichnet,

daß die Vorwahltemperaturen von Hand vorwählbar sind.

3. Vorrichtung nach Anspruch 1 oder 2,

dadurch gekennzeichnet,

daß das elektrisch steuerbare Ventil (29) ein Umschalt-Mischventil ist.

4. Vorrichtung nach Anspruch 2 oder 3,

dadurch gekennzeichnet,

daß der Speicher (42) eine Mehrzahl von aufeinander
abfolgenden Zeitintervallen zugeordneten, gesonderten Temperatureinstellelemente (51) aufweist.

5. Vorrichtung nach Anspruch 4,

dadurch gekennzeichnet,

daß die Temperatureinstellelemente (51) parallel nebeneinanderliegende Temperaturschieber sind.